(19) Europäisches Patentamt / European Patent Office / Office européen des brevets

(11) **EP 4 424 291 B1**

(12) **EUROPEAN PATENT SPECIFICATION**

(45) Date of publication and mention
of the grant of the patent:
**06.05.2026 Bulletin 2026/19**

(21) Application number: **23159813.7**

(22) Date of filing: **03.03.2023**

(51) International Patent Classification (IPC):
*A61F 13/49* (2006.01)     *A61F 13/56* (2006.01)

(52) Cooperative Patent Classification (CPC):
**A61F 13/49; A61L 15/60;** A61F 2013/530481;
A61F 2013/530671

(54) **MONOMER COMPOSITION FOR PREPARING SUPERABSORBENT POLYMER COMPRISED IN AN ABSORBENT ARTICLE**

MONOMERZUSAMMENSETZUNG ZUR HERSTELLUNG EINES SUPERABSORBIERENDEN POLYMERS IN EINEM SAUGFÄHIGEN ARTIKEL

COMPOSITION MONOMÈRE POUR PRÉPARER UN POLYMÈRE SUPERABSORBANT COMPRIS DANS UN ARTICLE ABSORBANT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**04.09.2024 Bulletin 2024/36**

(73) Proprietor: **The Procter & Gamble Company Cincinnati, OH 45202 (US)**

(72) Inventors:
- **SIMONYAN, Arsen**
  **65824 Schwalbach am Taunus (DE)**
- **BORGMANN, Carolin**
  **65824 Schwalbach am Taunus (DE)**
- **WON, TaeYoung**
  **17336 Seoul (KR)**

- **SOHN, Jung-min**
  **17336 Seoul (KR)**
- **RYU, Jihye**
  **Seoul 17336 (KR)**
- **HAN, Chang Hun**
  **Seoul 17336 (KR)**
- **KIM, Yun Soo**
  **Seoul 17336 (KR)**

(74) Representative: **P&G Patent Germany Procter & Gamble Service GmbH Sulzbacher Straße 40 65824 Schwalbach am Taunus (DE)**

(56) References cited:
EP-A1- 3 342 787     EP-A1- 3 391 960
US-A1- 2008 215 026

EP 4 424 291 B1

## Description

Technical Field

This invention relates to a monomer composition for preparing superabsorbent polymer, the superabsorbent polymer being comprised in an absorbent article.

Background of the Invention

Superabsorbent polymers (SAPs) are synthetic polymer materials that can absorb moisture of 500 to 1000 times of self-weight, began to be commercialized in absorbent articles, and currently, it is being widely used as water-holding material for soil, water stop material for civil engineering and architecture, sheets for raising seedling, freshness preservatives in the field of food circulation, fomentation material, and the like, besides hygienic goods such as paper diapers for children or sanitary pads, and the like.

Superabsorbent polymer is generally prepared by neutralizing water soluble unsaturated ethylene-based monomers with an alkali metal salt such as sodium salt, or a basic compound such as caustic soda, and then, adding a crosslinking agent, a polymerization initiator, and the like to polymerize the prepared monomer composition.

As a method for improving the absorption speed of superabsorbent polymer, a method of adding a blowing agent to conduct polymerization while bubbles are formed in the monomer composition, thereby forming a porous structure in superabsorbent polymer to increase the surface area, is known.

However, in the case of a preparation method of superabsorbent polymer using the existing blowing agent, bubbles generated by the blowing agent are lost before monomers are polymerized, and thus, sufficient pores cannot be formed in superabsorbent polymer. Thus, a method of reducing loss of bubbles using a bubble stabilizer together with a blowing agent has been suggested, but it has a problem with deterioration of surface tension of superabsorbent polymer due to the bubble stabilizer.

Therefore, there is a demand for development of superabsorbent polymer exhibiting rapid absorption speed without deterioration of properties such as surface tension, and the like.

EP3391960 relates to superabsorbent polymer particles which comprise clay platelets with edge modification and/or surface modification.

EP3342787 discloses a process to make continuous strand polymer comprising the following steps: a) providing a monomer mixture comprising water, a monomer, a crosslinker and an initiator; b) transporting the monomer mixture of step a) into a confining means; c) initiating polymerization of the monomer mixture in the confining means to make a continuous strand polymer; and d) emitting the continuous strand polymer from the confining means.

Technical Problem

It is an object of the invention to provide a monomer composition for preparing superabsorbent polymer that can prepare superabsorbent polymer having rapid absorption speed and excellent gel strength and wherein the superabsorbent polymer is subsequently incorporated in an absorbent article.

Technical Solution

There is provided a monomer composition for preparing superabsorbent polymer for incorporation in an absorbent article, the monomer composition comprising:

acrylic acid-based monomers having acid groups, at least a part of said acid groups being neutralized; a crosslinking agent; clay; a carbonate-based blowing agent; and a polymerization initiator, wherein the crosslinking agent, the clays and the carbonate-based blowing agent are used
in the amounts as specified by claim 1,
wherein, when irradiating heat and/or light to the monomer composition for preparing superabsorbent polymer to progress a polymerization reaction, a normalized gel point represented to by the following Formula 1 is 0.01 0.1:

[Formula 1]

$$\text{Normalized gel point} = \text{gel point(second)} / \text{total polymerization time(second)}$$

in the Formula 1, a gel point is a polymerization time at the intersection point of a storage modulus graph of the monomer composition for preparing superabsorbent polymer according to a polymerization time, and a loss modulus graph of the monomer composition for preparing superabsorbent polymer according to a polymerization time.

The absorbent article comprising the superabsorbent polymer made by using the monomer composition, may be a diaper or a pant.

The superabsorbent polymer may be incorporated into an absorbent core, the absorbent core being comprised by the absorbent article.

The absorbent core may be made by providing two nonwoven webs and incorporating the surface-crosslinked particulate water-absorbing resin between the two nonwoven webs.

The absorbent core, in between the two nonwoven webs, may comprise less than 20 weight-% of cellulose fibers, preferably less than 10 weight-% of cellulose fibers, and more preferably less than 5 weight-% of cellulose fibers.

In the absorbent core, the superabsorbent polymer may be adhesively immobilized in between the two nonwoven webs.

The absorbent article may comprise a topsheet, a backsheet and the absorbent core in between the topsheet and the backsheet.

There is also provided a method for preparing the monomer composition for preparing superabsorbent polymer, comprising steps of: mixing acrylic acid-based monomers having acid groups, at least a part of said acid groups being neutralized; a crosslinking agent; a carbonate-based blowing agent; and a polymerization initiator, and adding clay to the mixture and shear mixing at a stirring speed of 6,500 rpm or more.

There is further provided a polymer prepared from the monomer composition for preparing superabsorbent polymer, wherein, when the moisture content is 40 wt% to 80 wt%, gel strength is 40,000 Pa to 60,000 Pa.

Moreover, there is provided superabsorbent polymer prepared from the monomer composition for preparing superabsorbent polymer, wherein Performance Index (PI) represented by the following Mathematical Formula 1 is 2.35 or more, and T20 is less than 190 seconds:

[Calculation Formula 1]

$$\text{Performance Index (PI)} = (CRC/27) + (AUP/24) + (SFC/30) - (T20/140)$$

in the Calculation Formula 1,

CRC is centrifuge retention capacity(g/g) of superabsorbent polymer to 0.9 wt% sodium chloride aqueous solution for 30 seconds,

AUP is absorbency under 4826.33 Pa (0.7 psi) pressure(g/g) of superabsorbent polymer to 0.9 wt% sodium chloride aqueous solution for 1 hour,

SFC is saline flow conductivity of 0.685 wt% sodium chloride aqueous solution, and

T20 is a time(seconds) taken for 1g of superabsorbent polymer to absorb 20g of C12-14 alcohol ethoxylate aqueous solution under 2068.43 Pa (0.3 psi).

Also, there is provided a method for preparing superabsorbent polymer comprising steps of: irradiating heat and/or light to the monomer composition for preparing superabsorbent polymer, to polymerize, thus preparing hydrogel polymer; drying, grinding and classifying the hydrogel polymer to form base resin; and forming a surface crosslink layer on the surface of the base resin, in the presence of a surface crosslinking solution comprising a surface crosslinking agent and a solvent.

The superabsorbent polymer of the present invention is, subsequent to its making, incorporated into an absorbent article.

Effect of the Invention

The monomer composition for preparing superabsorbent polymer of the invention, wherein the superabsorbent polymer is subsequently incorporated into an absorbent article, has high gelation speed after initiation of polymerization, and has excellent bubble stabilizing effect, and thus, can minimize loss of bubbles generated from the blowing agent. Thus, superabsorbent polymer prepared from the monomer composition for preparing superabsorbent polymer of the invention has plural pores inside, and may exhibit excellent absorption speed.

And, since superabsorbent polymer prepared from the monomer composition for preparing superabsorbent polymer of the invention has high gel strength, and thus, maintains the shape well even after moisture absorption, it may exhibit excellent absorption performance in spite of external pressure.

Detailed Description of the Invention

The language and terms used herein are only to explain illustrative embodiments, and are not intended to limit the invention. A singular expression includes a plural expression thereof, unless it is expressly stated or obvious from the context that such is not intended. As used herein, the terms "comprise", "equipped" or "have", etc. are intended to designate the existence of practiced characteristic, number, step, constructional element or combinations thereof, and they are not intended to preclude the possibility of existence or addition of one or more other characteristics, numbers, steps, constructional elements or combinations thereof.

As used herein, 'clay' means a particle of a phyllosilicate mineral, or an aggregate of plurality of such particles, and a 'clay dispersion" means a dispersion in which the clay is dispersed in a solvent.

As used herein, (meth)acrylate is used to mean both acrylate and methacrylate.

As used herein, "base resin" or "base resin powder" refers to polymer polymerized from water soluble ethylene-based unsaturated monomers and dried and ground to particles or powders, wherein surface modification or surface crosslinking is not conducted.

Hereinafter, the invention will be explained in detail.

The monomer composition for preparing superabsorbent polymer, to be subsequently incorporated into an absorbent article, comprises: acrylic acid-based monomers having acid groups, at least a part of said acid groups being neutralized; a crosslinking agent; clay; a carbonate-based wherein the crosslinking agent, the clay and the blowing agent; and a polymerization initiator, carbonate-based blowing agent is used in amounts as defined by claim 1.

wherein, when irradiating heat and/or light to the monomer composition for preparing superabsorbent polymer to progress a polymerization reaction, a normalized gel point represented by the following Formula 1 meets 0.01 to 0.1:

[Formula 1]

Normalized gel point = gel point(second) / total polymerization time(second)

in the Formula 1, a gel point is a polymerization time at the intersection point of a storage modulus graph of the monomer composition for preparing superabsorbent polymer according to a polymerization time, and a loss modulus graph of the monomer composition for preparing superabsorbent polymer according to a polymerization time.

The normalized gel point of the monomer composition for preparing superabsorbent polymer may be 0.1 or less, or 0.09 or less, or 0.08 or less, or 0.06 or less, and 0.01 or more, or 0.02 or more. The measurement method of the gel point will be specifically explained in Examples later.

If polymer is formed from a monomer composition for preparing superabsorbent polymer, a liquid monomer composition turns into hydrogel polymer. Thus, a polymerization speed can be estimated by confirming a gel point, which is a time when the monomer composition is gelled. Wherein, by comparing the 'normalized gel point', which excludes the factors of a polymerization time affected by the amount of monomers, the amount of an initiator, a initial polymerization temperature, and/or other polymerization conditions, the polymerization speed of the monomer composition for preparing superabsorbent polymer can be evaluated.

The monomer composition for preparing superabsorbent polymer of the invention achieves polymerization more rapidly than the existing monomer composition, thereby minimizing loss of bubbles generated from the blowing agent, and thus, the absorption speed of prepared superabsorbent polymer can be improved even without excessively using a blowing agent.

However, if the normalized gel point is less than 0.01 and a polymerization speed is too rapid, is it likely that gelation may be progressed before the monomer composition is introduced into a polymerization reactor, and a problem of input pipe blocking may be generated. And, the viscosity of the monomer composition rapidly increases till the gel point, and if the gel point is less than 0.01, it may be difficult to mix the monomer composition, and thus, it may be difficult to form uniform polymer. And, if the normalized gel point is greater than 0.1, the above explained bubble loss decreasing effect may not be secured, and thus, the effect of improving the absorption speed of superabsorbent polymer may not be expected.

And, the monomer composition for preparing superabsorbent polymer, when heat and/or light is irradiated to progress a polymerization reaction, may exhibit an increase speed of gel strength(storage modulus) from the gel point to the time of completion of polymerization ($\Delta G'/s$, $\Delta G'$ denotes a difference between storage modulus at the time of completion of polymerization, and storage modulus at gel point, and s denotes a time from the gel point to the time of completion of polymerization), of 210 Pa/s or more.

Preferably, the increase speed of gel strength may be 215 Pa/s or more, or 220 Pa/s or more, or 230 Pa/s or more. Meanwhile, the upper limit of the increase speed of gel strength is not specifically limited, but for example, it may be 400 Pa/s or less, or 370 Pa/s or less, or 350 Pa/s or less. As such, the monomer composition for preparing superabsorbent polymer of the invention exhibits both rapid polymerization speed and high gel strength increase speed, and thus, can prepare polymer having excellent gel strength.

As such, since a gel strength increase speed as well as a gel point is improved, hydrogel polymer prepared from the monomer composition for preparing superabsorbent polymer, which is obtained immediately after the completion of polymerization, may have gel strength of 35,000 Pa or more, or 36,000 Pa or more, or 37,000 Pa or more, when the moisture content is 40 to 80 wt%, or 40 to 60 wt%. Meanwhile, the upper limit of the gel strength of the hydrogel polymer is not specifically limited, but for example, it may meet 60,000 Pa or less, or 58,000 Pa or less, or 55,000 Pa or less.

The monomer composition for preparing superabsorbent polymer comprises acrylic acid-based monomers; an alkali metal salt or an alkali compound capable of neutralizing the watersoluble ethylene-based unsaturated monomers; a crosslinking agent; clay; a carbonate-based blowing agent; and a polymerization initiator, thus meeting the above properties. Specifically, the monomer composition for preparing superabsorbent polymer of the invention comprises clay as an additive, and since such clay is uniformly dispersed in the monomer composition, the above explained properties may be exhibited.

The acrylic-based monomer is a compound represented by the following Chemical Formula 1:

$$[\text{Chemical Formula 1}] \quad R^1\text{-}COOM^1$$

R is a C2-5 alkyl group comprising unsaturated bond,

M' is a hydrogen atom, monovalent or divalent metal, an ammonium group or an organic amine salt.

Preferably, the monomer comprises one or more selected from the group consisting of acrylic acid, methacrylic acid, and monovalent metal salt, divalent metal salt, ammonium salt and organic amine salt thereof.

Wherein, the acrylic acid-based monomer has acid groups, and at least a part of the acid groups may be neutralized. Preferably, the monomers may be partially neutralized with alkali substances such as sodium hydroxide, potassium hydroxide, ammonium hydroxide, and the like before use. Wherein, the degree of neutralization of the acrylic acid-based monomers may be 40 to 95 mol%, or 40 to 80 mol%, or 45 to 75 mol%. The range of neutralization degree may be controlled according the final properties. However, if the degree of neutralization is too high, neutralized monomers may be extracted, and thus, it may be difficult to smoothly progress polymerization, and to the contrary, if the degree of neutralization is too low, absorption force of polymer may be significantly lowered, and the polymer may exhibit elastic rubber-like properties, which are difficult to handle.

The concentration of the acrylic acid-based monomers may be about 20 to about 60 wt%, preferably about 40 to about 50 wt%, based on the monomer composition comprising the raw materials of the superabsorbent polymer and a solvent, and it may be appropriately controlled considering a polymerization time and reaction conditions, and the like. However, if the concentration of the monomers is too low, yield of superabsorbent polymer may be low, thus causing a problem in terms of economical feasibility, and to the contrary, if the concentration is too high, a part of the monomers may be extracted, or grinding efficiency of hydrogel polymer may be low, thus causing process problems, and the properties of superabsorbent polymer may be deteriorated.

As the crosslinking agent, any compounds may be used as long as they enable introduction of crosslink during polymerization of the acrylic acid-based monomers. The crosslinking agent is also represented as an 'internal crosslinking agent' so as to distinguish it from a 'surface crosslinking agent' for surface crosslinking of superabsorbent polymer particles. Although crosslinking due to the crosslinking agent included in the monomer composition is progressed without division of the surface or inside, in case a surface crosslinking process of superabsorbent polymer particles obtained after polymerization, drying, grinding and classification of the monomer composition is progressed, the particle surface of the finally prepared superabsorbent polymer may consist of a structure crosslinked by the surface crosslinking agent, and the inside may consist of a structure crosslinked by the internal crosslinking agent.

Specifically, as the crosslinking agent included in the monomer composition, a crosslinking agent having one or more functional groups capable of reacting with the water soluble substituents of the acrylic acid-based monomers, and simultaneously having one or more ethylenic unsaturated groups; or a crosslinking agent having two or more functional groups capable of reacting with the water soluble substituents of the monomers and/or water soluble substituents formed by hydrolysis of the monomers, may be used.

As non-limiting examples, as the crosslinking agent, multifunctional crosslinking agents such as N,N'-methylenebisacrylamide, trimethylpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, or ethylene carbonate may be used alone or in combinations of two or more kinds, but it is not limited thereto.

Preferably, as the crosslinking agent, polyalkylene glycol di(meth)acrylate-based compounds such as polyethylene glycol (meth)acrylate, polyethylene glycol di(meth)acrylate, propylene glycol di(meth)acrylate, or polypropylene glycol (meth)acrylate may be used. When such a crosslinking agent is used, foaming by the carbonate-based blowing agent as described later may be easily progressed.

In the monomer composition, such a crosslinking agent is used in the content of 0.01 to 5 parts by weight, based on 100 parts by weight of the acrylic acid-based monomers. For example, the crosslinking agent may be used in the content of 0.01 parts by weight or more, 0.05 parts by weight or more, 0.1 parts by weight, or 0.15 parts by weight or more, and 5 parts by weight or less, 3 parts by weight or less, 2 parts by weight or less, 1 part by weight or less, or 0.7 parts by weight or less, based on 100 parts by weight of the acrylic acid-based monomers. If the content of the crosslinking agent is too low, crosslinking may not sufficiently occur, and thus, it may be difficult to realize strength above an appropriate level, and if the content of the crosslinking agent is too high, internal crosslinking density may increase, and thus, it may be difficult to realize desired centrifuge retention capacity.

And, the monomer composition comprises a polymerization initiator for initiating the polymerization reaction of the monomers. The polymerization initiator is not specifically limited as long as it is commonly used for the preparation of superabsorbent polymer.

Specifically, as the polymerization initiators, thermal polymerization initiators, red-ox pair initiators or photo polymerization initiators according to UV irradiation may be used according to a polymerization method. However, even in the case of photo polymerization, since a certain amount of heat is generated by UV irradiation, and the like, and heat is generated to some degree according to the progression of the exothermic polymerization reaction, a thermal polymerization initiator may be additionally included.

The photopolymerization initiator may be used without limitations in terms of its constructions, as long as it is a compound capable of forming radicals by light such as UV.

As the photo polymerization initiators, for example, one or more compounds selected from the group consisting of benzoin ether, dialkyl acetophenone, hydroxyl alkylketone, phenyl glyoxylate, benzyl dimethyl ketal, acyl phosphine and $\alpha$-aminoketone may be used. Among them, as specific examples of the acyl phosphine, lucirin TPO, i.e., 2,4,6-trimethyl-benzoyl-trimethyl phosphine oxide may be used. More various photo initiators are stated in Reinhold Schwalm, "UV Coatings: Basics, Recent Developments and New Application(Elsevier 2007)" p115, and are not limited to the above examples.

The photopolymerization initiator may be included in the concentration of about 0.001 to about 1.0 wt%, based on the monomer composition. If the concentration of the photopolymerization initiator is too low, a polymerization speed may become slow, and if the concentration of the photopolymerization initiator is too high, the molecular weight of superabsorbent polymer may be low and the properties may become non-uniform.

As the thermal polymerization initiator, one or more selected from the group consisting of persulfate-based initiators, azo-based initiators, hydrogen peroxide and ascorbic acid may be used. Specifically, as the examples of the persulfate-based initiators, sodium persulfate($Na_2S_2O_8$), potassium persulfate($K_2S_2O_8$), ammonium persulfate($(NH_4)_2S_2O_8$), and the like, may be mentioned, and as the examples of the azo-based initiators, 2,2-azobis(2-amidinopropane) dihydrochloride, 2,2-azobis-(N,N-dimethylene)isobutyramidine dihydrochloride, 2-(carbamoylazo)isobutylonitrile, 2,2-azobis[2-(2-imidazolin-2-yl)propane] dihydrochloride, 4,4-azobis-(4-cyanovaleric acid), and the like may be mentioned. More various thermal polymerization initiators are stated in Odian, "Principle of polymerization(Wiley, 1981)', p203, but the invention is not limited thereto.

The thermal polymerization initiator may be included in the content of about 0.001 to about 0.5 wt%, based on the monomer composition. If the concentration of the thermal polymerization initiator is too low, additional thermal polymerization may hardly occur, and thus, the effect resulting from the addition of the thermal polymerization initiator may be insignificant, and if the concentration of the thermal polymerization initiator is too high, the molecular weight of superabsorbent polymer may become small, and the properties may become non-uniform.

The red-ox pair initiators are materials causing oxidation-reduction reaction with each other in the monomer composition, and for example, potassium metabisulfite and sodium persulfate; or ascorbic acid and hydrogen peroxide may be used as the red-ox pair initiators.

Since the red-ox pair initiators cause the initiation of polymerization before photopolymerization due to radicals produced during an oxidation-reduction process, it is preferable to introduce in the monomer composition just before polymerization. The red-ox pair initiator (namely, each of oxidizing agent and reducing agent) may be used in the content of about 0.001 to about 0.5 wt%, based on the monomer composition.

The clay is included in the monomer composition so as to improve a polymerization speed and increase gel strength. As used herein, 'clay' means a clay particle having a nano size, or an aggregate of plurality of such particles.

As the clay, swellable or non-swellable clay may be used. The swellable clay is a layered organic substance having water absorption force, and montmorillonite, saponite, nontronite, laponite, beidellite, hectorite, vermiculite, magadiite, bentonite, and the like may be used, and as the non-swellable clay, kaolin, serpentine, mica, and the like may be used. One kind of the clay may be used alone, or two or more kinds of the clays may be used in combination.

As the clay, those having an average particle diameter of 0.025 μm or more, or 0.05 μm or more, or 0.1 μm or more, or 1 μm or more, and 10 μm or less, or 8 μm or less, or 5 μm or less, or 3 μm or less may be used. If the average particle diameter of the clay is less than 0.025 μm, the effect of improving gel strength of superabsorbent polymer may not be sufficiently obtained. If the average particle diameter of the clay is greater than 10 μm, due to the strong interlayer attraction force of clays, it may be difficult to form a uniform aqueous dispersion, and thus, transparency may not be secured after dispersion. The average particle diameter of the clay may be measured using a particle size analyzer, by Laser Diffraction and Dynamic Light Scattering.

The content of the clays is 0.1 parts by weight or more, or 0.2 parts by weight or more, or 0.25 parts by weight or more, and 10 parts by weight or less, or 7 parts by weight or less, or 5 parts by weight or less, or 3 parts by weight or less, or 1 part by weight or less, based on 100 parts by weight of the acrylic acid-based monomers. If the content of the clays is less than 0.1 parts by weight, based on 100 parts by weight of the acrylic acid-based monomers, the normalized gel point range as explained above may not be met. And, if the content of the clays is greater than 10 parts by weight, based on 100 parts by weight of the acrylic acid-based monomers, it may be difficult to uniformly disperse in the monomer composition, and the effect of improving the absorption speed of prepared superabsorbent polymer may not be obtained.

Meanwhile, in order to meet the above explained gel point property, clays are required to be uniformly dispersed in the monomer composition.

In this respect, it is preferred to use a clay that has been delaminated physically or chemically, or to delaminate the clay within the monomer composition.

Clay has a layered structure in which layers of small plates of silicate of about 1 nm thickness are stacked on top of each other due to strong van der Waals attraction, and the distance between each layer is about 1 nm. Delaminated clays are clays in which the silicate layers have been physically or chemically delaminated, i.e., spread apart so that the distance between layers exceeds 1 nm. The delaminated clays exhibit a more stable dispersion within the composition.

For example, clays that are surface-modified with a polymer dispersant comprising two or more functional groups selected from the group consisting of an amine group, a carbonyl group, and a hydroxyl group may be used as the clay. Such clay has remarkably improved dispersion stability as the layered structure is delaminated by the polymer dispersant, and thus, even if included in a composition for preparing superabsorbent polymer comprising an alkali metal salt or a basic compound, clay particles may be uniformly dispersed in the composition without aggregation or sedimentation.

Throughout the specification, 'two or more functional groups selected from the group consisting of an amine group, a carbonyl group, and a hydroxyl group' are used to include functional groups in which two or more kinds of an amine group, a carbonyl group and a hydroxyl group are combined, such as an amide group simultaneously comprising an amie group and a carbonyl group, a carboxyl group simultaneously comprising a carbonyl group and a hydroxyl group, and the like. For example, a 'polymer dispersant comprising an amine group and a carbonyl group' may comprise an amine group and a carbonyl group respectively in the molecule, or may comprise an amide group in which an amine group and a carbonyl group are combined.

The polymer dispersant comprising the functional groups may bond to the surface of clay due to unshared electrons of nitrogen and oxygen atoms, and in the composition for preparing superabsorbent polymer of high ion concentration, due to resonance, charge may be induced in the polymer. Due to such properties, the polymer dispersant may improve dispersibility of clay by delaminating its layered structure. Moreover, the polymer dispersant does not cause discoloration of superabsorbent polymer or deteriorate properties, and thus, it may be suitably used for the preparation of superabsorbent polymer.

In this respect, it may be preferable that the polymer dispersant comprises an amine group and a carbonyl group; or a carbonyl group and a hydroxyl group. More preferably, the polymer dispersant may comprise an amide group and/or a carboxyl group.

As specific examples of the polymer dispersant, one or more selected from the group consisting of polyvinylpyrrolidone, polyacrylamide, and polyacrylic acid may be mentioned. Preferably, polyvinylpyrrolidone may be used as the polymer dispersant.

Although the molecular weight of the polymer dispersant is not specifically limited, for example, those having weight average molecular weight of 2,500 g/mol or more, or 5,000 g/mol or more, or 10,000 g/mol or more, and 200,000 g/mol or less, or 100,000 g/mol or less, or 40,000 g/mol may be used. If the weight average molecular weight of the polymer dispersant is less than 2,500 g/mol, when the polymer dispersant bonds to the surface of clay, it may be difficult to sufficiently secure distances between the silicate layers of clay, and thus, there may be problems in terms of dispersibility and long-term stability, and if it is greater than 200,000 g/mol, there may be a process difficulty, and it may not be effective for surface modification of clay, and thus, it is preferable to meet the above ranges.

The polymer dispersant may be used in the content of greater than 20 parts by weight to 200 parts by weight or less, based on 100 parts by weight of clays, and preferably in the content of 23 parts by weight or more, or 25 parts by weight or more, and 150 parts by weight or less, or 100 parts by weight or less, so as to secure dispersibility of clays.

If the content of the polymer dispersant is 20 parts by weight or less, based on 100 parts by weight of clays, a degree of surface modification of clay may be insufficient, and thus, interlayer distance between clays may not be sufficiently widened. Thus, dispersion stability of clay may not be sufficient, and thus, clay particles may be easily aggregated. And, if the content of the polymer dispersant is greater than 200 parts by weight, based on 100 parts by weight of clays, the polymer dispersant may lower the property improvement effect that can be provided by clay, and thus, the properties of superabsorbent polymer may be deteriorated.

The clay surface-modified with the polymer dispersant may be prepared by adding clays and a polymer dispersant to a solvent such as water, lower alcohol such as ethanol, glycol, and the like, and stirring. And, the clay surface-modified with the polymer dispersant may be added to the monomer composition for preparing superabsorbent polymer in the form of a dispersion in which the clays are dispersed in a solvent. As the solvent for preparing the clay dispersion, solvents used for the monomers for preparing superabsorbent polymer may be used, and the amount of the solvent used may be appropriately controlled considering the kinds and amounts of clay and polymer dispersant, and use of a clay dispersion, and the like.

For example, the dispersion comprising clays surface-modified with the polymer dispersant may comprise clays in the content of 1 part by weight or more, or 3 parts by weight or more, and 20 parts by weight or less, or 10 parts by weight or less, or 8 parts by weight or less, in 100 parts by weight of the dispersion.

Meanwhile, in case non-surface-modified clay is used, a clay dispersion can be shear mixed and added to the monomer composition, or alternatively, the clay can be added to the monomer composition and then shear mixed. Such shear mixing can physically delaminate the clay, thereby allowing the clay to be more uniformly and stably dispersed in the monomer composition.

Regarding solvents and the content of clay in the dispersion for preparing a clay dispersion comprising non-surface-modified clay, reference may be made to a description of the dispersion comprising clays surface-modified with the polymer dispersant.

The shear mixing may be conducted using an apparatus such as in-line high shear mixer, a high shear batch mixer or a homogenizer, and the like.

The shear mixing may be performed at a stirring speed of 6,500 rpm or more, or 9,000 rpm or more, or 12,000 rpm or more. Although the upper limit of the stirring speed is not specifically limited, for example, it may be 50,000 rpm or less, or 40,000 rpm or less, or 30,000 rpm or less.

By shear mixing for at least 10 seconds or more, preferably 20 seconds or more, or 30 seconds or more under such a stirring speed, clays may be physically delaminated. Wherein, it the shear mixing time is too lengthened, heat may be generated due to shear force, and thus, it is preferable that the shear mixing is conducted for 3 minutes or less, or 1 minute or less.

Meanwhile, in case that the clay is introduced into the monomer composition and then shear mixing is performed, in order to achieve more uniform dispersion degree, the non-surface-modified clay may be added to the monomer composition in the form of an aqueous dispersion. The clay aqueous dispersion may be prepared by mixing water and clays at a stirring speed of 6,500 rpm or more, or 9,000 rpm or more, or 12,000 rpm or more, using the above-explained shear mixing apparatus. Although the upper limit of the stirring speed during shear mixing is not specifically limited, for example, it may be 50,000 rpm or less, or 40,000 rpm or less, or 30,000 rpm or less. By mixing for 10 seconds or more, or 20 seconds or more, or 30 seconds or more, and 5 minutes or less, or 3 minutes or less, under the above stirring speed, the aqueous dispersion of clays may be prepared.

Wherein, it may be appropriate for securing of uniform dispersibility that the concentration of the clay aqueous dispersion, namely, the content of clays in 100 parts by weight of the clay aqueous dispersion is 0.5 parts by weight or more, or 1.0 parts by weight or more, or 2.0 parts by weight or more, and 5.0 parts by weight or less, or 4.0 parts by weight or less

As explained above, by using clays surface-modified with a polymer dispersant, or by physically delaminating non-surface-modified clays, clays may be uniformly dispersed in the monomer composition, and the monomer composition prepared may meet the above explained gel point property.

Meanwhile, whether or not clays are 'uniformly dispersed' in the monomer composition may be judged through whether or not layer separation of the monomer composition occurs, when the clays are mixed and then left for 1 minute. Namely, in case layer separation does not occur even after the monomer composition is left for 1 minute, it can be predicted that the clays are uniformly dispersed in the monomer composition, and thus, the composition may meet the above-explained gel point property.

The carbonate-based blowing agent is foamed during polymerization, thus functioning for forming pores in hydrogel polymer to increase the surface area, and for example, one or more selected from the group consisting of sodium bicarbonate, sodium carbonate, potassium bicarbonate, potassium carbonate, calcium bicarbonate, calcium bicarbonate, magnesiumbicarbonate and magnesium carbonate may be used.

The carbonate-based blowing agent used in the content of 0.005 to 1 part by weight, based on 100 parts by weight of the acrylic acid-based monomers. If the content of the blowing agent is less than 0.005 parts by weight, the function as a blowing agent may be insignificant, and if the content of the blowing agent is greater than 1 part by weight, due to too many pores in crosslinked polymer, gel strength of prepared superabsorbent polymer may be lowered, and density may decrease, thus causing problems in terms of distribution and storage. For example, the carbonate-based blowing agent may be used in the content of 0.01 parts by weight or more, 0.05 parts by weight or more, and 0.5 parts by weight or less, 0.3 parts by weight or less, or 0.2 parts by weight or less, based on 100 parts by weight of the acrylic acid-based monomers.

When preparing the monomer composition, a surfactant commonly used as a bubble stabilizer, such as an alkyl sulfate compound and a polyoxyethylene alkylether compound, may not be used. For example, in the step 1 and step 2, cationic surfactant such as quaternary ammonium compounds, such as dodecyltrimethylammonium chloride, dodecyltrimethylammonium bromide, and the like; anionic surfactant such as alkyl sulfate compounds, such as sodium dodecyl sulfate, ammonium lauryl sulfate, sodium lauryl ether sulfate, or sodium myreth sulfate, and the like; or non-ionic surfactant such as alkyl ether sulfate compounds, such as polyoxyethylene lauryl ether, and the like, may not be used. Thereby, decrease in the surface tension of superabsorbent polymer due to use of the surfactant may be prevented.

The monomer composition may further comprise additives such as a thickener, a plasticizer, a preservative, an antioxidant, and the like, as necessary.

And, the monomer composition comprising monomers may be in the state of a solution dissolved in a solvent such as water, and the solid content in the monomer composition of a solution state, namely, the concentration of monomers, crosslinking agent and polymerization initiator may be appropriately controlled considering polymerization time and reaction conditions, and the like. For example, the solid content in the monomer composition may be 10 to 80 wt%, or 15 to 60 wt%, or 30 to 50 wt%.

In case the monomer composition has the above range of solid content, due to gelation effect appeared in the polymerization reaction of a high concentration aqueous solution, there is no need to remove unreacted monomers after polymerization, and simultaneously, it may be favorable for controlling of grinding efficiency during grinding of polymer as described later.

Wherein, a solvent that can be used is not limited as long as it can dissolve the above explained raw materials, and for example, water, ethanol, ethyleneglycol, diethyleneglycol, triethyleneglycol, 1,4-butanediol, propyleneglycol, ethyleneglycol monobutyl ether, propyleneglycol monomethyl ether, propyleneglycol monomethyl ether acetate, methylethylketone, acetone, methylamylketone, cyclohexanone, cyclopentanone, diethyleneglycol monomethyl ether, diethyleneglycol ethyl ether, toluene, xylene, butyrolactone, carbitol, methyl celosolve acetate and N,N-dimethylacetamide or mixtures thereof may be used.

Meanwhile, there is provided a method for preparing superabsorbent polymer, comprising steps of: irradiating heat and/or light to the monomer composition for preparing superabsorbent polymer to polymerize, thus preparing hydrogel polymer; drying, grinding and classifying the hydrogel polymer to form base resin; and forming a surface crosslink layer on the surface of the base resin, in the presence of a surface crosslinking solution comprising a surface crosslinking agent and a solvent. The method comprises the further step of incorporating the superabsorbent polymer into an absorbent article.

A method for polymerizing the monomer composition for preparing superabsorbent polymer is not specifically limited. The polymerization method is largely divided into thermal polymerization and photopolymerization according to a polymerization energy source, and commonly, thermal polymerization may be progressed in a reactor equipped with a stirring axis such as a kneader, and photopolymerization may be progressed in a reactor equipped with a movable conveyor belt or in a flat-bottom container, but the above explained polymerization method is no more than one example, and the invention is not limited thereto.

For example, hydrogel polymer may be obtained by introducing the monomer composition into a reactor equipped with a stirring axis such as a kneader as explained above, and supplying hot air or heating the reactor to progress thermal polymerization. Wherein, the hydrogel polymer discharged to the outlet of the reactor may be in the size of a few centimeters to a few millimeters according to the shape of the stirring axis equipped in the reactor. Specifically, the size of obtained hydrogel polymer may vary according to the concentration of the introduced monomer composition and the introduction speed, and the like, and commonly, hydrogel polymer with particle diameter of 2 to 50 mm may be obtained.

And, in case photopolymerization of the monomer composition is progressed in a reactor equipped with a movable conveyer belt as explained above, hydrogel polymer obtained may be commonly a hydrogel polymer sheet having a width of the belt. Wherein, the thickness of the sheet may vary according to the concentration of the introduced monomer composition and the introduction speed, but it is preferable that the monomer composition is supplied so as to obtain a polymer sheet with a thickness of about to 0.5 to about 5 cm. In case the monomer composition is fed such that the thickness of a polymer sheet may become too thin, production efficiency may be low, and if the thickness of a polymer sheet is greater than 5cm, due to too thick thickness, polymerization may not uniformly occur over the entire thickness.

The moisture content of hydrogel polymer thus obtained may be commonly 40 to 80 wt%. Throughout the specification, the "moisture content" is the content of moisture occupied based on the total weight of hydrogel polymer, and it means a value obtained by subtracting the weight of polymer of a dry state from the weight of hydrogel polymer. Specifically, it is defined as a value calculated by measuring the weight loss according to moisture evaporation in the polymer while raising the temperature of polymer through infrared heating to dry. Wherein, the temperature is raised from room temperature to about 180°C and then maintained at 180°C, and the total drying time is 20 minutes including a temperature raising step of 5 minutes.

Next, a step of drying and classifying the hydrogel polymer to form base resin in the form of powders is conducted. In order to increase the efficiency of the drying step, a coarsely grinding step may be further conducted before drying, as necessary.

Wherein, grinders that can be used are not limited in terms of the constructions, but specifically, one selected from the group consisting of a vertical pulverizer, a turbo cutter, a turbo grinder, a rotary cutter mill, a cutter mill, a disc mill, a shred crusher, a crusher, a chopper, a disc cutter may be used, but the invention is not limited thereto.

Wherein, the grinding may be conducted such that the particle diameter of the polymer may become about 2 to 10 mm. Grinding to a particle diameter less than 2 mm would not be technologically easy due to the high moisture content of hydrogel polymer, and may cause aggregation between ground particles. Meanwhile, if ground to a particle diameter greater than 10 mm, the effect of increasing the efficiency of a drying step conducted later may be insignificant.

The polymer ground as described above, or polymer immediately after polymerization, not having passed through the grinding step, is dried. Wherein, the drying temperature of the drying step may be about 150 to about 250°C. If the drying temperature is less than 150°C, drying time may too lengthen, and the properties of the finally prepared superabsorbent polymer may be deteriorated, and if the drying temperature is greater than 250°C, only the surface of polymer may be dried, and thus, fine particles may be generated in the grinding process conducted later, and the properties of the finally prepared superabsorbent polymer may be deteriorated. Thus, the drying may be preferably progressed at a temperature of about 150 to about 200°C, more preferably at a temperature of about 160 to about 180°C.

Meanwhile, the drying may be progressed for about 20 minutes to about 90 minutes considering process efficiency, but the drying time is not limited thereto.

And, the drying method is not limited as long as it is commonly used as a drying process of hydrogel polymer. Specifically, the drying may be progressed by hot air supply, infrared irradiation, microwave irradiation or UV irradiation, and the like. The moisture content of polymer after the drying step may be about 0.1 to about 5wt%.

Next, a step of grinding dried polymer obtained through the drying step is conducted.

Polymer powders obtained after the grinding step, namely, base resin may have particle diameter of about 150 to about 850$\mu$m. As a grinder used to grind to such a particle diameter, specifically, a ball mill, a pin mill, a hammer mill, a screw mill, a roll mill, a disc mill or a jog mill, a

And, in order to manage the properties of superabsorbent polymer powders finally productized after the grinding step, base resin obtained after grinding is classified according to particle diameter. Preferably, polymers having particle diameters of about 150 to about 850$\mu$m are classified, and only the base resin having such particle diameter may be passed through a surface crosslinking reaction step. Such a particle diameter may be measured according to European Disposables and Nonwovens Association (EDANA) standard EDANA WSP 220.3 method.

Next, a step of additionally crosslinking the surface of the base resin in the presence of a surface crosslinking agent to form a surface crosslink layer is conducted. Through the step, superabsorbent polymer in which a surface crosslink layer formed on the surface of the base resin, more specifically, on at least a part of each surface of base resin particles, is provided.

Surface crosslinking is a step of increasing the crosslinking density around the surfaces of superabsorbent polymer particles in relation to the crosslinking density inside the particles. In general, a surface crosslinking agent is applied on the surfaces of superabsorbent polymer particles. Thus, the reaction occurs on the surfaces of superabsorbent polymer particles, which improves crosslinkability of the surfaces of particles, without substantially affecting the inside of the particles. Thus, surface crosslinked superabsorbent polymer particles have higher crosslinking density around surface than inside.

As the surface crosslinking agent, those previously used in the preparation of superabsorbent polymer may be used without specific limitations. For example, the surface crosslinking agent may comprise one or more polyols selected from the group consisting of ethylene glycol, propylene glycol, 1,3-ptopanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol and glycerol; one or more carbonate-based compounds selected from the group consisting of ethylene carbonate, propylene carbonate and glycerol carbonate; epoxy compounds such as ethylene glycol diglycidyl ether, and the like; oxazolidinone compounds such as 2-oxazolidinone, and the like; polyamine compounds; oxazoline compounds; mono-, di- or polyoxazolidinone compounds; or cyclic urea compounds; and the like. Specifically, as the surface crosslinking agent, one or more, or 2 or more, or 3 or more kinds of the above-described surface crosslinking agents may be used. For example, ethylene carbonate may be used as the surface crosslinking agent.

A method of mixing the surface crosslinking agent with base resin is not limited. The surface crosslinking agent and base resin powder may be put in a reactor and mixed, a surface crosslinking agent may be sprayed to base resin powders, or base resin and surface crosslinking agent may be continuously fed to a continuously operated mixer and mixed, and the like.

When adding the surface crosslinking agent, it may be added in the form of a surface crosslinking solution by mixing water together. In case water is added, the surface crosslinking agent may be uniformly dispersed in the polymer. Wherein, it is preferable that water is added at a ratio of about 1 part by weight to about 10 parts by weight, based on 100 parts by weight of the base resin, so as to induce uniform dispersion of the surface crosslinking agent, prevent agglomeration of polymer powders, and simultaneously optimize surface penetration depth of the surface crosslinking agent.

By heating base resin to which the surface crosslinking solution comprising a surface crosslinking agent and a solvent is added at a temperature of about 100 to about 150°C, preferably about 110 to about 140°C for about 15 to about 80 minutes, preferably about 20 to about 70 minutes, a surface crosslinking reaction may be conducted.

A temperature rise means for the surface crosslinking is not specifically limited, and a heat transfer medium may be supplied, or a heat source may be directly supplied for heating. Wherein, as the heat transfer medium that can be used, steam, hot air, temperature-raised fluid such as hot oil may be used, and the temperature of supplied heat transfer medium may be appropriately selected considering the heat transfer medium, temperature rise means and target temperature. Meanwhile, as the heat source directly supplied, electric heating or gas heating may be mentioned, but the invention is not limited thereto.

The monomer composition for preparing superabsorbent polymer has high gelation speed after initiation of polymerization, thus minimizing loss of bubbles generated from the blowing agent. Thus, the monomer composition for preparing superabsorbent polymer can provide superabsorbent polymer exhibiting excellent absorption speed.

Specifically, superabsorbent polymer prepared from the monomer composition for preparing superabsorbent polymer may meet Performance Index (PI) represented by the following Mathematical Formula 1 of 2.35 or more, and T20 of less than 190 seconds:

[Calculation Formula 1]

$$\text{Performance Index (PI)} = (CRC/27) + (AUP/24) + (SFC/30) - (T20/140)$$

in the Calculation Formula 1,

CRC is centrifuge retention capacity(g/g) of superabsorbent polymer to 0.9 wt% sodium chloride aqueous solution for 30 seconds,
AUP is absorbency under pressure(g/g) of superabsorbent polymer to 0.9 wt% sodium chloride aqueous solution for 1 hour,
SFC is saline flow conductivity($\cdot 10^{-7} m^3 \cdot s/g$) of 0.685 wt% sodium chloride aqueous solution, and
T20 is a time(seconds) taken for 1g of superabsorbent polymer to absorb 20g of C12-14 alcohol ethoxylate aqueous solution under 2068.43 Pa (0.3 psi).

The measurement methods of the CRC, AUP, SFC, and T20 will be specifically explained in Examples below.

Preferably, PI of the superabsorbent polymer may be 2.37 or more, 2.40 or more, or 2.41 or more, and 4.0 or less, or 3.0 or less. And, T20 of the superabsorbent polymer may be 180 seconds or less, 160 seconds or less, 140 seconds or less, 130 seconds or less, or 125 seconds or less, and 100 seconds or more.

And, superabsorbent polymer prepared from the monomer composition for preparing superabsorbent polymer of the invention has high gel strength, and maintains the shape well even after moisture absorption, and thus, it may exhibit excellent absorption performance in spite of external pressure.

Absorbent articles

The superabsorbent polymer of the present disclosure is incorporated in an absorbent article, such as in an absorbent core comprised by the absorbent article.

"Absorbent article" refers to devices that absorb and contain body exudates, particularly urine and other water-containing liquids, and, more specifically, refers to devices that are placed against or in proximity to the body of the wearer to absorb and contain the various exudates discharged from the body. Absorbent articles may include diapers (for babies and infants as well as for adult incontinence), pants (for babies and infants as well as for adult incontinence). As used herein, the term "exudates" includes, but is not limited to, urine, blood, vaginal discharges, sweat and fecal matter. Preferred absorbent articles of the present invention are disposable absorbent articles, more preferably disposable diapers, disposable pants and disposable absorbent inserts.

"Absorbent core" is used herein to refer to a structure intended to be disposed between a topsheet and backsheet of an absorbent article for absorbing and storing liquid received by the absorbent article.

"Disposable" is used in its ordinary sense to mean an article that is disposed or discarded after a limited number of usage events over varying lengths of time, for example, less than 10 events, less than 5 events, or less than 2 events. If the disposable absorbent article is a diaper, a pant, absorbent insert, sanitary napkin, sanitary pad or wet wipe for personal hygiene use, the disposable absorbent article is most often intended to be disposed after single use. The used and disposed absorbent article may or may not be subsequently recycled. As used herein, the terms 'absorbent article', 'pant' and 'diaper' always also refer to disposable absorbent articles, disposable pants (as well as disposable absorbent pants), and disposable diapers.

"Diaper" and "pant" refers to an absorbent article generally worn by babies, infants and incontinent persons about the lower torso so as to encircle the waist and legs of the wearer and that is specifically adapted to receive and contain urinary and fecal waste. In a pant, as used herein, the longitudinal edges of the first and second waist region are attached to each other to a pre-form waist opening and leg openings. A pant is placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (e.g., seam, weld, adhesive, cohesive bond, fastener, etc.). A pant may be pre-formed anywhere along the circumference of the article (e.g., side fastened, front waist fastened). In a diaper, the waist opening and leg openings are only formed when the diaper is applied onto a wearer by (releasable) attaching the longitudinal edges of the first and second waist region to each other on both sides by a suitable fastening system.

Absorbent articles may each include a topsheet, a backsheet, an absorbent core and optionally an acquisition-distribution system. The absorbent core is placed between the backsheet and the topsheet, the optional acquisition-distribution system is typically placed between the absorbent core and the topsheet.

A superabsorbent polymer of the present disclosure may be incorporated in the absorbent core of the absorbent article. The absorbent core may or may not include other absorbent material in addition to the superabsorbent polymer, such as non-crosslinked cellulose fibers (pulp fibers). The absorbent core may include at least 60 mass%, at least 75 mass%, at least 85 mass%, at least 95 mass%, or at least 98 mass%, or 100 mass% of the superabsorbent polymer disclosed herein.

A diaper or pant may also include elasticized leg cuffs and barrier leg cuffs, which provide improved containment of liquids and other body exudates especially in the area of the leg openings. Usually each of the leg cuffs and barrier cuffs includes one or more elastic strings.

A "feminine care absorbent article" is a personal care product used by women during menstruation to absorb and retain menses, vaginal discharge, and matters from other bodily functions related to vulva. Feminine care absorbent articles include pantiliners and sanitary napkins.

Examples

**Comparative Example 1**

(1) Preparation of monomer composition for preparing superabsorbent polymer

In a 3L glass container equipped with a stirrer and a thermometer, 450 g of acrylic acid, 3 g of PEGDA 400(polyethylene glycol diacrylate 400) internal crosslinking agent, and 0.04 g of diphenyl(2,4,6-trimethylbenzoyl)-phosphine oxide photoinitiator were added and dissolved, and then, 580 g of 31.5% sodium hydroxide solution was added to prepare a monomer mixture (neutralization degree: 70 mol%; solid content: 41 wt%).

To the monomer mixture, sodium bicarbonate(SBC) was added as a carbonate-based blowing agent in the content of 0.1 parts by weight, based on 100 parts by weight of acrylic acid, thus preparing a monomer composition for preparing superabsorbent polymer.

(2) Preparation of superabsorbent polymer

A part (86.6g) of the monomer composition for preparing superabsorbent polymer prepared in (1) was put in a rheometer (TA Instruments, ARES) equipped with UV-accessory, and irradiated by UV to conduct UV polymerization for 180 seconds. Specifically, the polymerization temperature was maintained at 60°C, UV was irradiated for 60 seconds(irradiation amount: 100 mW/cm$^2$), and the reaction was progressed additionally for 120 seconds.

Wherein, storage modulus(G') and loss modulus(G") of the monomer composition were measured from the time of initiation of polymerization, and plotted to polymerization time. The moisture content of hydrogel polymer obtained after the polymerization reaction was measured, and the result was confirmed to be 48%.

The remainder of the monomer composition for preparing superabsorbent polymer, which was not added to the rheometer, was introduced in a polymerization chamber of which temperature was controlled to 60°C, in which UV light source exists at the upper part, and then, UV was irradiated for 60 seconds, and the reaction was progressed additionally for 120 seconds to obtain hydrogel polymer. The prepared hydrogel polymer was cut to a size of about 5 cm x 5 cm, and then, introduced into a meat chopper to grind the polymer, thus obtaining hydrogel crumb having a size of 1 mm to 10 mm. And then, the crumb was dried in an oven capable of transferring airflow up and down. The crumb was uniformly dried by flowing hot air of 180°C or more from the lower part to the upper part for 15 minutes, and flowing again from the upper part to the lower part for 15 minutes, and the drying was progressed such that the moisture content of dried body after drying became 1% or less. After drying, it was ground with a grinder, and classified to select those having size of 150 to 850 $\mu$m, thus preparing base resin.

To 100 parts by weight of the above prepared base resin, a surface crosslinking solution comprising, based on 100 parts by weight of the base resin, 1.5 parts by weight of ethylene carbonate and 6 parts by weight of water was sprayed, and stirred and mixed at room temperature such that the surface crosslinking solution was uniformly distributed on the surface of the base resin powders. Subsequently, the base resin powders mixed with the surface crosslinking solution were put in a surface crosslinking reactor and a surface crosslinking reaction was progressed.

In the surface crosslinking reactor, it was confirmed that the temperature of the base resin powder was gradually raised from the initial temperature around 80°C, and it was operated such that the maximum reaction temperature of 190°C was reached after 30 minutes. After reaching the maximum reaction temperature, it was additionally reacted for 15 minutes, and then, the finally prepared superabsorbent polymer sample was taken. After the surface crosslinking process, it was classified with ASTM standard sieve to prepare superabsorbent polymer having particle diameter of 150$\mu$m to 850 $\mu$m.

**Example 1**

In a 3L glass container equipped with a stirrer and a thermometer, 450 g of acrylic acid, 3 g of PEGDA 400(polyethylene glycol diacrylate 400) internal crosslinking agent, and 0.04 g of diphenyl(2,4,6-trimethylbenzoyl)-phosphine oxide photoinitiator were added and dissolved, and then, 580g of 31.5% sodium hydroxide solution was added to prepare a monomer mixture (neutralization degree: 70 mol%; solid content: 41 wt%).

To the monomer mixture, an aqueous dispersion of kaolin (average particle diameter 0.6 $\mu$m) was added such that the content of kaolin became 0.25 parts by weight, based on 100 parts by weight of acrylic acid, and mixed at 6500 rpm for 3 minutes using a homogenizer (IKA, Ultra-turrax) to disperse. The aqueous dispersion of kaolin was prepared by mixing 96 g of water and 4 g of kaolin at 6500 rpm for 30 seconds using a homogenizer (IKA, Ultra-turrax).

And then, sodium bicarbonate(SBC) was added and mixed in the content of 0.1 parts by weight, based on 100 parts by weight of acrylic acid, and just before initiation of polymerization, 0.12 g of hydrogen peroxide($H_2O_2$), 0.04 g of ascorbic acid and 2 g of sodium persulfate were added to prepare a monomer composition for preparing superabsorbent polymer.

Using the monomer composition, superabsorbent polymer was prepared by the same method as (2) of Comparative Example 1.

**Example 2**

A monomer composition for preparing superabsorbent polymer and superabsorbent polymer was prepared by the same method as Example 1, except that kaolin(average particle diameter 0.6 $\mu$m) was added in the content of 0.5 parts by weight, based on 100 parts by weight of acrylic acid.

**Example 3**

A monomer composition for preparing superabsorbent polymer and superabsorbent polymer was prepared by the same method as Example 1, except that kaolin(average particle diameter 0.6 $\mu$m) was added in the content of 1 part by weight, based on 100 parts by weight of acrylic acid.

**Example 4**

A monomer composition for preparing superabsorbent polymer and superabsorbent polymer was prepared by the same method as Example 1, except that bentonite(average particle diameter 0.8 $\mu$m) was added in the content of 0.25 parts by weight, based on 100 parts by weight of acrylic acid.

**Example 5**

A monomer composition for preparing superabsorbent polymer and superabsorbent polymer was prepared by the same method as Example 1, except that bentonite(average particle diameter 0.8 $\mu$m) was added in the content of 0.5 parts by weight, based on 100 parts by weight of acrylic acid.

**Example 6**

A monomer composition for preparing superabsorbent polymer and superabsorbent polymer was prepared by the same method as Example 1, except that bentonite(average particle diameter 0.8 μm) was added in the content of 1 part by weight, based on 100 parts by weight of acrylic acid.

**Comparative Example 2**

Superabsorbent polymer was prepared by the same method as Comparative Example 1, except that the polymerization temperature was 50°C.

**Comparative Example 3**

A monomer composition for preparing superabsorbent polymer and superabsorbent polymer were prepared by the same method as Comparative Example 1, except that 0.12 g of hydrogen peroxide($H_2O_2$) and 0.04 g of ascorbic acid were additionally added when preparing the monomer composition.

**Comparative Example 4**

A monomer composition for preparing superabsorbent polymer and superabsorbent polymer was prepared by the same method as Comparative Example 1, except that sodium bicarbonate(SBC) was added in the content of 0.2 parts by weight, based on 100 parts by weight of acrylic acid, as a blowing agent, when preparing the monomer composition.

**Comparative Example 5**

A monomer composition for preparing superabsorbent polymer and superabsorbent polymer was prepared by the same method as Comparative Example 1, except that sodium bicarbonate(SBC) was added in the content of 0.3 parts by weight, based on 100 parts by weight of acrylic acid, as a blowing agent, when preparing the monomer composition.

**Experimental Example 1: Property evaluation of monomer composition for preparing superabsorbent polymer**

From the G' plot and G" plot obtained during the polymerization of each monomer composition for preparing superabsorbent polymer of Examples and Comparative Examples, a gel point(GP), a normalized GP, G' at GP, the final G' of polymer after completion of polymerization, and increase speed of G'(ΔG'/s) from the gel point till the time of completion of polymerization were calculated, and the results were shown in the following Table 1.
The gel point is a time(s) at the intersection point of the G' plot and G" plot, and the normalized gel point was calculated according to the following Formula 1.

[Formula 1]

Normalized gel point = gel point(sec) / total polymerization time(sec)

**Experimental Example 2: Evaluation of the properties of superabsorbent polymer**

(1) Vortex time of base resin

The vortex time of each superabsorbent polymer of Examples and Comparative Examples was measured as follows. For the measurement of vortex time, superabsorbent polymer classified with #30-50 sieve was used.

① First, in a flat-bottomed beaker with a capacity of 100 mL, 50 mL of 0.9% brine was introduced using 100 mL mass cylinder.
② Next, the beaker was laid at the center a magnetic stirrer, and then, a circular magnetic bar(diameter 8 mm, length 30 mm) was put in the beaker.
③ Thereafter, the stirrer was operated such that the magnetic bar stirs at 600 rpm, and the lowest part of vortex generated by stirring was made to touch on the magnetic bar.

④ After confirming that the temperature of brine in the beaker became 24.0°C, 2±0.01 g of superabsorbent polymer sample was introduced, and simultaneously, a stop watch was operated, and a time taken until vortex disappeared and the liquid surface became completely horizontal was measured, which was designated as vortex time.

**(2) Centrifuge Retention Capacity (CRC)**

Centrifuge retention capacity (CRC) was measured according to European Disposables and Nonwovens Association(EDANA) standard EDANA WSP 241.3. $W_0(g$, about 0.2g) of the superabsorbent polymer was uniformly put in an envelope made of non-woven fabric and the envelope was sealed, and then, the envelope was dip in a saline solution of 0.9 wt% sodium chloride aqueous solution. After 30 minutes, the envelope was drained at 250G for 3 minutes using a centrifuge, and then, the weight $W_2(g)$ of the envelope was measured. Meanwhile, the same operation was conducted without using superabsorbent polymer, and then, the weight $W_1(g)$ at that time was measured. Using the weights thus obtained, CRC(g/g) was calculated according to the following Calculation Formula 2 to confirm centrifuge retention capacity.

$$[\text{Calculation Formula 2}]$$

$$CRC(g/g) = \{[W_2(g) - W_1(g) - W_0(g)]/W_0(g)\}$$

**(3) Absorbing under Pressure (AUP)**

For the superabsorbent polymers prepared in Examples and Comparative Examples, Absorbency under Pressure(AUP) was measured according to the method of European Disposables and Nonwovens Association) standard EDANA WSP 242.3-10.

First, on the bottom of a plastic cylinder having an inner diameter of 60 mm, a 400 mesh wire netting made of stainless steel was installed. And, $W_0(g$, about 0.90g) of each superabsorbent polymer obtained in Examples and Comparative Examples was uniformly sprayed on the wire netting under temperature of 23±2°C and humidity of 45%, and a piston capable of uniformly applying a load of 4.83 kPa (0.7 psi) was added, wherein the piston had an outer diameter slightly smaller than 60 mm and had no gap with the inner wall of the cylinder, and the up and down movement was not hindered. At this time, the weight $W_3(g)$ of the apparatus was measured.

Inside a petri dish having a diameter of 150mm, a glass filter having a diameter of 125mm and a thickness of 5mm was laid, and a saline solution of 0.90 wt% sodium chloride was poured to the same level as the upper side of the glass filter. And, the above apparatus was put on the glass filter, and the liquid was absorbed under load for 1 hour. After 1 hour, the measurement apparatus was lifted, and the weight $W_4(g)$ was measured.

Using the measured weights, AUP(g/g) was calculated according to the following Calculation Formula 3 to confirm absorbency under pressure.

$$[\text{Calculation Formula 3}]$$

$$AUP(g/g) = [W_4(g) - W_3(g)]/ W_0(g)$$

In the Calculation Formula 3,

$W_0(g)$ is the initial weight(g) of superabsorbent polymer,

$W_3(g)$ is the sum of the weight of superabsorbent polymer and the weight of the apparatus capable of applying load to the superabsorbent polymer, and

$W_4(g)$ is the sum of the weight of superabsorbent polymer and the weight of the apparatus capable of applying load to the superabsorbent polymer, after absorbing a saline solution in the superabsorbent polymer under load (4826.33 Pa (0.7 psi)) for 1 hour.

**(3) Saline Flow Conductivity(SFC)**

It was measured and calculated according to the method disclosed in column 54 to column 59 of US Registered Patent No. 5562646.

**(4) T20**

9 g of sodium chloride and 0.1 g of Lorodac (main component: linear C12-14 alcohol ethoxylate, CAS# 68439-50-9) were dissolved in 1L of distilled water to form an aqueous solution, and a time taken for 1g of the superabsorbent polymer to absorb 20 g of the aqueous solution under load of 2068.43 Pa (0.3 psi) was calculated and measured. Specific measurement method of T20 was described in detail in pages 13-18 of EP2535027A1.

**(5) Performance Index (PI)**

Using the CRC(g/g), AUP(g/g), SFC($\cdot 10^{-7}$cm$^3$·s/g), and T20(sec) measured by the above methods, Performance Index (PI, no unit) was calculated through the following Calculation Formula 1.

[Calculation Formula 1]

$$\text{Performance Index (PI)} = (\text{CRC}/27) + (\text{AUP}/24) + (\text{SFC}/30) - (\text{T20}/140)$$

[Table 1]

| | Kind of clay | Content of clay* | GP(s) | Normal ized GP | G' at GP' (Pa) | Final G' (Pa) / moisture content (wt%) | ΔG'/s | Vortex time of Base resin(s) |
|---|---|---|---|---|---|---|---|---|
| Comparative Example 1 | - | - | 20 | 0.11 | 203 | 33350/48 | 207 | 60 |
| Example 1 | Kaolin | 0.25 | 15 | 0.08 | 200 | 38420/45 | 232 | 42 |
| Example 2 | Kaolin | 0.5 | 13 | 0.07 | 195 | 40160/47 | 239 | 44 |
| Example 3 | Kaolin | 1 | 14 | 0.08 | 190 | 41040/50 | 246 | 45 |
| Example 4 | Bentonite | 0.25 | 14 | 0.08 | 20 | 43150/51 | 260 | 40 |
| Example 5 | Bentonite | 0.5 | 9 | 0.05 | 2 | 48180/49 | 282 | 41 |
| Example 6 | Bentonite | 1 | 4 | 0.02 | 2 | 50130/48 | 285 | 42 |
| Comparative Example 2 | - | - | 19 | 0.11 | 210 | 31100/53 | 192 | 48 |
| Comparative Example 3 | - | - | 21 | 0.12 | 150 | 19570/51 | 185 | 59 |
| * parts by weight, based on 100 parts by weight of acrylic acid | | | | | | | | |

[Table 2]

| | CRC(g/g) | AUP(g/g) | SFC ($\cdot 10^{-7}$cm$^3$·s/g) | T20(sec) | PI |
|---|---|---|---|---|---|
| Comparative Example 1 | 28.8 | 24.9 | 36 | 138 | 2.32 |
| Example 1 | 28.5 | 25.9 | 34 | 120 | 2.41 |
| Example 2 | 28.8 | 26.3 | 35 | 119 | 2.48 |
| Example 3 | 28.7 | 26.4 | 34 | 122 | 2.42 |
| Example 4 | 29.4 | 25.9 | 36 | 103 | 2.63 |
| Example 5 | 29.5 | 26.3 | 34 | 105 | 2.57 |
| Example 6 | 29.0 | 26.6 | 35 | 109 | 2.57 |
| Comparative Example 2 | 28.8 | 24.7 | 34 | 131 | 2.29 |
| Comparative Example 3 | 29.1 | 24.5 | 33 | 134 | 2.24 |
| Comparative Example 4 | 28.4 | 24.8 | 34 | 128 | 2.30 |
| Comparative Example 5 | 28.1 | 24.7 | 31 | 120 | 2.25 |

**Claims**

1. Method of making an absorbent article comprising superabsorbent polymer, wherein the superabsorbent polymer is prepared by using a monomer composition, the monomer composition comprising:

   acrylic acid-based monomers having acid groups, at least a part of said acid groups being neutralized; a crosslinking agent; clay; a carbonate-based blowing agent; and a polymerization initiator,
   wherein the crosslinking agent is used in the content of 0.01 to 5 parts by weight, based on 100 parts by weight of the acrylic acid-based monomers; and
   wherein the content of the clays is 0.1 parts by weight or more, and 10 parts by weight or less, based on 100 parts by weight of the acrylic acid-based monomers; and
   wherein the carbonate-based blowing agent is used in the content of 0.005 to 1 part by weight, based on 100 parts by weight of the acrylic acid-based monomers;
   wherein, when irradiating heat and/or light to the monomer composition for preparing superabsorbent polymer to progress a polymerization reaction, a normalized gel point represented by the following Formula 1 is 0.01 to 0.1:

   [Formula 1]

   $$\text{Normalized gel point} = \text{gel point(second)} / \text{total polymerization time(second)}$$

   in the Formula 1, a gel point is a polymerization time at the intersection point of a storage modulus graph of the monomer composition for preparing superabsorbent polymer according to a polymerization time, and a loss modulus graph of the monomer composition for preparing superabsorbent polymer according to a polymerization time.

2. The method of claim 1, wherein, when irradiating heat and/or light to the monomer composition for preparing superabsorbent polymer to progress a polymerization reaction, increase speed of gel strength from the gel point to the time of completion of polymerization is 210 Pa/s or more.

3. The method of claim 1 or 2,, wherein the clay is included in the monomer composition in the content of 0.1 to 5 parts by weight, based on 100 parts by weight of the acrylic acid-based monomers.

4. The method of any of the preceding claims, wherein, in the monomer composition for preparing the superabsorbent polymer, the clay has an average particle diameter of 0.025 $\mu$m to 10 $\mu$m.

5. The method of any of the preceding claims, wherein, in the monomer composition for preparing the superabsorbent polymer, the surface of the clay is modified with a polymer dispersant comprising 2 or more functional groups selected from the group consisting of an amine group, a carbonyl group and a hydroxyl group.

6. The method of any of the preceding claims, wherein, in the monomer composition for preparing the superabsorbent polymer, the polymer dispersant is one or more selected from the group consisting of polyvinyl pyrrolidone, polyacrylamide, and polyacrylic acid.

7. The method of any of the preceding claims, wherein the method comprises the further step of incorporating the superabsorbent polymer in the absorbent article.

8. A method for making an absorbent article, the method comprising the step of making the monomer composition of claim 1 for preparing superabsorbent polymer, the method comprising the steps of:

   mixing the acrylic acid-based monomers having acid groups, at least a part of said acid groups being neutralized; the crosslinking agent; the carbonate-based blowing agent; and
   the polymerization initiator; and
   adding the clay to the mixture, and shear mixing at a stirring speed of 6,500 rpm or more, wherein the method comprises the further step of incorporating the superabsorbent polymer prepared by the monomer composition in an absorbent article.

9. Method of making the absorbent article of claim 8, wherein a polymer is prepared from the monomer composition for preparing the superabsorbent polymer, wherein, the polymer has a gel strength of from 35,000 Pa to 60,000 Pa when the moisture content is 40 wt% to 80 wt%.

10. Absorbent article comprising the superabsorbent polymer prepared from the monomer composition of any of claims 1 to 7, wherein

The Performance Index (PI) of the superabsorbent polymer, represented by the following Calculation Formula 1, is 2.35 or more, and T20 is less than 190 seconds:

[Calculation Formula 1]

$$\text{Performance Index (PI)} = (CRC/27) + (AUP/24) + (SFC/30) - (T20/140)$$

in the Calculation Formula 1,
CRC is centrifuge retention capacity(g/g) of superabsorbent polymer to 0.9 wt% sodium chloride aqueous solution for 30 seconds,
AUP is absorbency under 4826.33 Pa (0.7 psi) pressure(g/g) of the superabsorbent polymer to 0.9 wt% sodium chloride aqueous solution for 1 hour,
SFC of the superabsorbent polymer is saline flow conductivity($\cdot 10\text{-}7cm3\cdot s/g$) of 0.685 wt% sodium chloride aqueous solution, and
T20 is a time(seconds) taken for 1g of the superabsorbent polymer to absorb 20g of C12-14 alcohol ethoxylate aqueous solution under 0.3 psi.

11. A method for making an absorbent article, the absorbent article comprising superabsorbent polymer, the method comprising the steps of:

irradiating heat and/or light to a monomer composition of claim 1 for preparing superabsorbent polymer to polymerize, thus preparing hydrogel polymer,
drying, grinding and classifying the hydrogel polymer to form base resin; and
forming a surface crosslink layer on the surface of the base resin, in the presence of a surface crosslinking solution comprising a surface crosslinking agent and a solvent, wherein the method further comprises the step of incorporating the superabsorbent polymer in an absorbent article.

12. The method of claim 11, wherein the absorbent article is a diaper or a pant.

13. The method of claim 11 or 12, wherein, the superabsorbent polymer is incorporated into an absorbent core, the absorbent core being comprised by the absorbent article.

14. The method of claim 13, wherein the absorbent core is made by providing two nonwoven webs and incorporating the surface-crosslinked particulate water-absorbing resin between the two nonwoven webs.

15. The method of claim 14, wherein the absorbent core, in between the two nonwoven webs, comprises less than 20 weight-% of cellulose fibers, preferably less than 10 weight-% of cellulose fibers, and more preferably less than 5 weight-% of cellulose fibers.

16. The method of claim 14 or 15, wherein the method comprises the further step of adhesively immobilizing the superabsorbent polymer in between the two nonwoven webs.

17. The method of any of claims 13 to 16, wherein the method comprises the further steps of:

- providing a topsheet
- providing a backsheet
- providing the absorbent core in between the topsheet and the backsheet.

**Patentansprüche**

1. Verfahren zum Erzeugen eines Absorptionsartikels, umfassend ein Superabsorber-Polymer, wobei das Superabsorber-Polymer durch Verwenden einer Monomerzusammensetzung hergestellt wird, die Monomerzusammensetzung umfassend:

   Monomere auf Acrylsäurebasis, die Säuregruppen aufweisen, wobei wenigstens ein Teil der Säuregruppen neutralisiert ist; ein Vernetzungsmittel; Ton; ein Treibmittel auf Carbonatbasis; und einen Polymerisationsinitiator, wobei das Vernetzungsmittel in dem Gehalt von 0,01 bis 5 Gewichtsteilen, basierend auf 100 Gewichtsteilen der Monomere auf Acrylsäurebasis, verwendet wird; und
   wobei der Gehalt der Tone 0,1 Gewichtsteile oder mehr und 10 Gewichtsteile oder weniger, basierend auf 100 Gewichtsteilen der Monomere auf Acrylsäurebasis, beträgt; und
   wobei das Treibmittel auf Carbonatbasis in einem Gehalt von 0,005 bis 1 Gewichtsteil, basierend auf 100 Gewichtsteilen der Monomere auf Acrylsäurebasis, verwendet wird;
   wobei, bei einem Bestrahlen der Monomerzusammensetzung mit Wärme und/oder Licht zum Herstellen des Superabsorber-Polymers, um eine Polymerisationsreaktion fortschreiten zu lassen, ein normalisierter Gelpunkt, dargestellt durch die folgende Formel 1, 0,01 bis 0,1 beträgt:

   [Formel 1]

   $$\text{Normalisierter Gelpunkt} = \text{Gelpunkt(Sekunde)} / \text{Gesamtpolymerisationszeit(Sekunde)}$$

   in der Formel 1, wobei ein Gelpunkt eine Polymerisationszeit an dem Schnittpunkt eines Speichermodulgraphen der Monomerzusammensetzung zum Herstellen des Superabsorber-Polymers nach einer Polymerisationszeit und eines Verlustmodulgraphen der Monomerzusammensetzung zum Herstellen des superabsorbierendes Polymers nach einer Polymerisationszeit ist.

2. Verfahren nach Anspruch 1, wobei, bei dem Bestrahlen der Monomerzusammensetzung mit Wärme und/oder Licht zum Herstellen des Superabsorber-Polymers, um eine Polymerisationsreaktion fortschreiten zu lassen, eine Erhöhungsgeschwindigkeit einer Gelstärke von dem Gelpunkt bis zu dem Zeitpunkt einer Vollendung einer Polymerisation 210 Pa/s oder mehr beträgt.

3. Verfahren nach Anspruch 1 oder 2, wobei der Ton in der Monomerzusammensetzung in dem Gehalt von 0,1 bis 5 Gewichtsteilen, basierend auf 100 Gewichtsteilen der Monomere auf Acrylsäurebasis, enthalten ist.

4. Verfahren nach einem der vorstehenden Ansprüche, wobei, in der Monomerzusammensetzung zum Herstellen des Superabsorber-Polymers, der Ton einen durchschnittlichen Teilchendurchmesser von 0,025 μm bis 10 μm aufweist.

5. Verfahren nach einem der vorstehenden Ansprüche, wobei, in der Monomerzusammensetzung zum Herstellen des Superabsorber-Polymers, die Oberfläche des Tons mit einem Polymerdispersant modifiziert ist, umfassend 2 oder mehr Funktionsgruppen, ausgewählt aus der Gruppe bestehend aus einer Amingruppe, einer Carbonylgruppe und einer Hydroxylgruppe.

6. Verfahren nach einem der vorstehenden Ansprüche, wobei, in der Monomerzusammensetzung zum Herstellen des Superabsorber-Polymers, das Polymerdispersant eines oder mehrere, ausgewählt aus der Gruppe bestehend aus Polyvinylpyrrolidon, Polyacrylamid und Polyacrylsäure, ist.

7. Verfahren nach einem der vorstehenden Ansprüche, wobei das Verfahren den weiteren Schritt eines Beimischens des Superabsorber-Polymers in den Absorptionsartikel umfasst.

8. Verfahren zum Erzeugen eines Absorptionsartikels, das Verfahren umfassend den Schritt des Erzeugens der Monomerzusammensetzung nach Anspruch 1 zum Herstellen des Superabsorber-Polymers, das Verfahren umfassend die Schritte:

   Mischen der Monomere auf Acrylsäurebasis, die Säuregruppen aufweisen, wobei wenigstens ein Teil der Säuregruppen neutralisiert ist; des Vernetzungsmittels; des Treibmittels auf Carbonatbasis; und des Polymerisationsinitiators; und

Hinzugeben des Tons zu der Mischung und Schubmischen bei einer Rührgeschwindigkeit von 6.500 U/min oder mehr,

wobei das Verfahren den weiteren Schritt des Beimischens des Superabsorber-Polymers, das durch die Monomerzusammensetzung hergestellt ist, in einen Absorptionsartikel umfasst.

9. Verfahren zum Erzeugen des Absorptionsartikels nach Anspruch 8, wobei ein Polymer aus der Monomerzusammensetzung zum Herstellen des Superabsorber-Polymers hergestellt wird, wobei das Polymer eine Gelfestigkeit von 35.000 Pa bis 60.000 Pa aufweist, wenn der Feuchtigkeitsgehalt 40 Gew.-% bis 80 Gew.-% beträgt.

10. Absorptionsartikel, umfassend das Superabsorber-Polymer, das aus der Monomerzusammensetzung nach einem der Ansprüche 1 bis 7 hergestellt ist, wobei

der Güteindex (PI) des Superabsorber-Polymers, dargestellt durch die folgende Berechnungsformel 1, 2,35 oder mehr beträgt, und T20 weniger als 190 Sekunden beträgt:

[Berechnungsformel 1]

$$\text{Güteindex (PI)} = (CRC/27) + (AUP/24) + (SFC/30) - (T20/140),$$

in der Berechnungsformel 1,
CRC eine Zentrifugenretentionskapazität(g/g) des Superabsorber-Polymers gegenüber 0,9 Gew.-% einer wässrigen Natriumchloridlösung für 30 Sekunden ist,
AUP ein Absorptionsvermögen unter 4826,33 Pa (0,7 psi) Druck(g/g) des Superabsorber-Polymers gegenüber 0,9 Gew.-% der wässrigen Natriumchloridlösung für 1 Stunde ist,
SFC des Superabsorber-Polymers eine Flüssigkeitsweiterleitung($\cdot 10$-7 cm3 s/g) gegenüber 0,685 Gew.-% der wässrigen Natriumchloridlösung ist, und
T20 eine Zeit(Sekunden) ist, die 1 g des Superabsorber-Polymers benötigt, um 20 g einer wässrigen C12-14-Alkoholethoxylatlösung unter 0,3 psi zu absorbieren.

11. Verfahren zum Erzeugen eines Absorptionsartikels, der Absorptionsartikel umfassend das Superabsorber-Polymer, das Verfahren umfassend die Schritte:

Bestrahlen einer Monomerzusammensetzung nach Anspruch 1 mit Wärme und/oder Licht zum Herstellen des Superabsorber-Polymers zum Polymerisieren und damit Herstellen eines Hydrogelpolymers,
Trocknen, Mahlen und Klassifizieren des Hydrogelpolymers, um ein Basisharz zu formen; und
Formen einer Oberflächenvernetzungsschicht auf der Oberfläche des Basisharzes unter Vorhandensein einer Oberflächenvernetzungslösung, umfassend ein Oberflächenvernetzungsmittel und ein Lösemittel, wobei das Verfahren ferner den Schritt des Beimischens des Superabsorber-Polymers in einen Absorptionsartikel umfasst.

12. Verfahren nach Anspruch 11, wobei der Absorptionsartikel eine Windel oder ein Höschen ist.

13. Verfahren nach Anspruch 11 oder 12, wobei das Superabsorber-Polymer in einen Absorptionskern beigemischt wird, wobei der Absorptionskern durch den Absorptionsartikel umfasst ist.

14. Verfahren nach Anspruch 13, wobei der Absorptionskern durch Bereitstellen von zwei Vliesbahnen und Beimischen des oberflächenvernetzten teilchenförmigen wasserabsorbierenden Harzes zwischen den zwei Vliesbahnen erzeugt wird.

15. Verfahren nach Anspruch 14, wobei der Absorptionskern zwischen den zwei Vliesbahnen weniger als 20 Gew.-% Cellulosefasern, vorzugsweise weniger als 10 Gew.-% Cellulosefasern und mehr bevorzugt weniger als 5 Gew.-% Cellulosefasern, umfasst.

16. Verfahren nach Anspruch 14 oder 15, wobei das Verfahren den weiteren Schritt eines Immobilisierens des Superabsorber-Polymers mittels Klebstoff zwischen den zwei Vliesbahnen umfasst.

17. Verfahren nach einem der Ansprüche 13 bis 16, wobei das Verfahren die weiteren Schritte umfasst:

- Bereitstellen einer Oberschicht
- Bereitstellen einer Unterschicht
- Bereitstellen des Absorptionskerns zwischen der Oberschicht und der Unterschicht.

**Revendications**

1. Procédé de fabrication d'un article absorbant comprenant un polymère superabsorbant, dans lequel le polymère superabsorbant est préparé en utilisant une composition monomère, la composition monomère comprenant :

   des monomères à base d'acide acrylique ayant des groupes acides, au moins une partie desdits groupes acides étant neutralisée ; un agent de réticulation ; de l'argile ; un agent gonflant à base de carbonate ; et un initiateur de polymérisation,
   dans lequel l'agent de réticulation est utilisé à une teneur de 0,01 à 5 parties en poids, sur la base de 100 parties en poids des monomères à base d'acide acrylique ; et
   dans lequel la teneur des argiles est de 0,1 partie en poids ou plus, et de 10 parties en poids ou moins, sur la base de 100 parties en poids des monomères à base d'acide acrylique ; et
   dans lequel l'agent gonflant à base de carbonate est utilisé à la teneur de 0,005 à 1 partie en poids, sur la base de 100 parties en poids des monomères à base d'acide acrylique ;
   dans lequel, lors de l'irradiation de chaleur et/ou de lumière sur la composition monomère pour préparer le polymère superabsorbant afin de faire progresser une réaction de polymérisation, un point de gel normalisé représenté par la formule 1 suivante va de 0,01 à 0,1 :

   [Formule 1]

$$\text{Point de gel normalisé} = \text{point de gel (seconde)/temps de polymérisation}$$

   total (seconde) dans la Formule 1, un point de gel est un temps de polymérisation au point d'intersection d'un graphique de module de conservation de la composition monomère pour la préparation du polymère superabsorbant en fonction d'un temps de polymérisation, et d'un graphique de module de perte de la composition monomère pour la préparation du polymère superabsorbant en fonction d'un temps de polymérisation.

2. Procédé selon la revendication 1, dans lequel, lors d'irradiation de chaleur et/ou de lumière sur la composition monomère pour préparer le polymère superabsorbant pour faire progresser une réaction de polymérisation, la vitesse d'augmentation de la résistance du gel depuis le point de gel jusqu'au moment d'achèvement de la polymérisation est de 210 Pa/s ou plus.

3. Procédé selon la revendication 1 ou 2, dans lequel l'argile est incluse dans la composition monomère à la teneur de 0,1 à 5 parties en poids, sur la base de 100 parties en poids des monomères à base d'acide acrylique.

4. Procédé selon l'une quelconque des revendications précédentes, dans lequel, dans la composition monomère pour la préparation du polymère superabsorbant, l'argile a un diamètre moyen de particule de 0,025 $\mu$m à 10 $\mu$m.

5. Procédé selon l'une quelconque des revendications précédentes, dans lequel, dans la composition de monomères pour la préparation du polymère superabsorbant, la surface de l'argile est modifiée avec un dispersant polymère comprenant 2 groupes fonctionnels ou plus choisis dans le groupe constitué d'un groupe amine, d'un groupe carbonyle et d'un groupe hydroxyle.

6. Procédé selon l'une quelconque des revendications précédentes, dans lequel, dans la composition monomère pour la préparation du polymère superabsorbant, le dispersant polymère est un ou plusieurs choisis dans le groupe constitué de polyvinylpyrrolidone, polyacrylamide, et acide polyacrylique.

7. Procédé selon l'une quelconque des revendications précédentes, dans lequel le procédé comprend l'étape supplémentaire consistant à incorporer le polymère superabsorbant dans l'article absorbant.

8. Procédé de fabrication d'un article absorbant, le procédé comprenant l'étape consistant à fabriquer la composition monomère selon la revendication 1 pour la préparation d'un polymère superabsorbant, le procédé comprenant les étapes consistant à :

mélanger les monomères à base d'acide acrylique ayant des groupes acides, au moins une partie desdits groupes acides étant neutralisée ; l'agent de réticulation ; l'agent gonflant à base de carbonate ; et l'initiateur de polymérisation ; et

ajouter l'argile au mélange, et mélanger par cisaillement à une vitesse d'agitation de 6 500 tr/min ou plus, dans lequel le procédé comprend l'étape supplémentaire consistant à incorporer le polymère superabsorbant préparé par la composition monomère dans un article absorbant.

9. Procédé de fabrication de l'article absorbant selon la revendication 8, dans lequel un polymère est préparé à partir de la composition de monomères pour préparer le polymère superabsorbant, dans lequel le polymère a une résistance de gel allant de 35 000 Pa à 60 000 Pa lorsque la teneur en humidité est de 40 % en poids à 80 % en poids.

10. Article absorbant comprenant le polymère superabsorbant préparé à partir de la composition monomère selon l'une quelconque des revendications 1 à 7, dans lequel

l'indice de performance (IP) du polymère superabsorbant, représenté par la formule de calcul 1 suivante, est de 2,35 ou plus, et T20 est inférieur à 190 secondes :

[Formule de calcul 1]

Indice de performance (IP) = (CRC/27) + (AUP/24) + (SFC/30) - (T20/140)

dans la formule de calcul 1,

CRC est la capacité de rétention centrifuge (g/g) du polymère superabsorbant pour une solution aqueuse de chlorure de sodium à 0,9 % en poids pendant 30 secondes,

AUP est la capacité d'absorption sous une pression de 4 826,33 Pa (0,7 psi) (g/g) du polymère superabsorbant pour une solution aqueuse de chlorure de sodium à 0,9 % en poids pendant 1 heure,

SFC du polymère superabsorbant est la conductivité de flux salin (·10 à 7 cm3·s/g) d'une solution aqueuse de chlorure de sodium à 0,685 % en poids, et

T20 est un temps (secondes) nécessaire pour que 1 g du polymère superabsorbant absorbe 20 g d'une solution aqueuse d'éthoxylate d'alcool en C12-14 sous 0,3 psi.

11. Procédé de fabrication d'un article absorbant, l'article absorbant comprenant un polymère superabsorbant, le procédé comprenant les étapes consistant à :

irradier de la chaleur et/ou de la lumière sur une composition monomère selon la revendication 1 pour préparer un polymère superabsorbant à polymériser, préparant ainsi un polymère hydrogel,

sécher, broyer et classer le polymère hydrogel pour former une résine de base ; et

former une couche de réticulation de surface sur la surface de la résine de base, en présence d'une solution de réticulation de surface comprenant un agent de réticulation de surface et un solvant, dans lequel le procédé comprend en outre l'étape consistant à incorporer le polymère superabsorbant dans un article absorbant.

12. Procédé selon la revendication 11, dans lequel l'article absorbant est une couche ou une culotte.

13. Procédé selon la revendication 11 ou 12, dans lequel le polymère superabsorbant est incorporé dans une âme absorbante, l'âme absorbante étant comprise dans l'article absorbant.

14. Procédé selon la revendication 13, dans lequel l'âme absorbante est fabriquée en fournissant deux bandes non tissées et en incorporant la résine particulaire absorbant l'eau réticulée en surface entre les deux bandes non tissées.

15. Procédé selon la revendication 14, dans lequel l'âme absorbante, entre les deux bandes non tissées, comprend moins de 20 % en poids de fibres de cellulose, de préférence moins de 10 % en poids de fibres de cellulose, et plus préférablement moins de 5 % en poids de fibres de cellulose.

16. Procédé selon la revendication 14 ou 15, dans lequel le procédé comprend l'étape supplémentaire consistant à immobiliser par adhésif le polymère superabsorbant entre les deux bandes non tissées.

**17.** Procédé selon l'une quelconque des revendications 13 à 16, dans lequel le procédé comprend les étapes supplémentaires consistant à :

- fournir une feuille de dessus
- fournir une feuille de fond
- fournir une âme absorbante entre la feuille de dessus et la feuille de fond.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3391960 A
- EP 3342787 A
- US 5562646 A
- EP 2535027 A1

**Non-patent literature cited in the description**

- **REINHOLD SCHWALM**. UV Coatings: Basics, Recent Developments and New Application. Elsevier, 2007, 115
- **ODIAN**. Principle of polymerization. Wiley, 1981, 203
- *CHEMICAL ABSTRACTS*, 68439-50-9